# EUROPEAN PATENT SPECIFICATION

(11) **EP 2 982 346 B1**
(45) Date of publication and mention of the grant of the patent: **22.05.2019**
(21) Application number: 13880916.5
(22) Date of filing: 01.04.2013
(51) Int. Cl.: A61F 2/82, A61F 2/958

(54) **INDWELLING OBJECT DELIVERY SYSTEM**
SYSTEM ZUR AUSGABE EINES EINDRINGUNGSOBJEKTS
SYSTÈME DE MISE EN PLACE À DEMEURE D'UN IMPLANT

(43) Date of publication of application: 10.02.2016
(73) Proprietor: Terumo Kabushiki Kaisha, Tokyo 151-0072 (JP)
(72) Inventor: KAWASHIMA, Yoshio, Fujinomiya-shi Shizuoka 418-0015 (JP)
(74) Representative: Casalonga
(86) International application number: PCT/JP2013/059851
(87) International publication number: WO 2014/162398

(56) References cited:
- EP-A1- 0 365 993
- EP-A1- 1 393 771
- EP-A1- 2 198 805
- WO-A1-2007/034639
- US-A- 5 643 278
- US-A1- 2002 120 322

## Description

### Technical Field

The present invention relates to a delivery system for a living body indwelling member which is used in order to deliver the living body indwelling member caused to indwell a living body, for example, such as a stent or the like, through a living body lumen such as blood vessels, biliary ducts, bronchial tubes, esophagi, urethrae and the like.

### Background Art

In recent years, in order to treat myocardinal infarction or angina pectoris, a method has been used in which a stent (living body indwelling member) is caused to indwell a lesion (stenosis) of a coronary artery so as to secure a space inside the coronary artery. In some cases, the same method has also been used in order to improve the stenosis appearing in other blood vessels, biliary ducts, bronchial tubes, esophagi, urethrae, and other living body lumens. The stent is classified into a balloon-expandable stent and a self-expandable stent, depending on functions and methods for stent indwelling.

In the balloon-expandable stent, the stent itself has no expandable function. In order to cause the stent to indwell a target area, a balloon catheter is used. The balloon catheter generally includes an elongated shaft portion and a balloon which is disposed on a distal side of the shaft portion and which is dilatable in a radial direction. The stent is mounted on an outer surface of the deflated balloon. When the stent is caused to indwell the target area, the balloon having the stent mounted thereon is caused to dilate after the balloon reaches a target area inside a body via thin living body lumens. In this manner, a dilating force of the balloon plastically deforms and expands the stent, and the stent is fixed by coming into close contact with an inner surface of the target area.

In this catheter, an X-ray contrast marker is generally attached to the shaft portion on the proximal side of the stent so that a position of the stent is visible when X-ray fluoroscopy is used (for example, refer to PTL 1).

### Citation List

### Patent Literature

PTL 1: Pamphlet of International Publication No.. US 5,643,278 discloses a stent delivery device with a radiopaque spring having a variable flexibility.

### Summary of Invention

### Technical Problem

However, in the balloon catheter disclosed in PTL 1, a contrast marker is disposed at a position where the contrast marker does not overlap the stent at an axial position of the shaft portion. Consequently, the catheter is kinked between the hard contrast marker and the stent or on the proximal side of the contrast marker, thereby resulting in poor insertion operability. In some cases, there is a possibility that the stent may be partially expanded and may slip out therefrom due to the kink.

In addition, a part on the distal side of the hard contrast marker is disposed at the position where the contrast marker overlaps the stent at the axial position, and a part on the proximal side of the contrast marker is disposed at the position where the contrast marker does not overlap the stent at the axial position. In this manner, a configuration is adopted in which a part of the contrast marker enters the stent. Accordingly, it is possible to suppress the kink between the hard contrast marker and the stent. However, even in this case, the kink of the catheter probably occurs on the proximal side of the contrast marker.

The present invention is made in order to solve the above-described problem, and an object thereof is to provide a delivery system for a living body indwelling member which can suppress kink probably occurring in an end portion of a contrast marker, and which can improve insertion operability and safety.

### Solution to Problem

In order to achieve the above-described object, a delivery system for a living body indwelling member according to the present invention includes a shaft portion that extends in a long length, a living body indwelling member that is disposed on a distal side of the shaft portion, and that is caused to indwell a living body, and a contrast marker that is disposed on at least one side of a proximal side or a distal side of the living body indwelling member, and that has contrasting property. The contrast marker is arranged up to a position where the contrast marker does not overlap the living body indwelling member in a separating direction away from the living body indwelling member from a position where the contrast marker overlaps the living body indwelling member at an axial position of the shaft portion, or from a position where the contrast marker coincides with an edge portion of the living body indwelling member. Bending rigidity of the contrast marker on the separating direction side is smaller than bending rigidity of the contrast marker on a side where the contrast marker comes close to the living body indwelling member.

### Advantageous Effects of Invention

According to a delivery system for a living body indwelling member which is configured as described above, a contrast marker is arranged up to a position where the contrast marker does not overlap the living body indwelling member in a separating direction away from the living body indwelling member from a position where the contrast marker overlaps the living body indwelling member, or from a position where the contrast marker coincides with an edge portion of the living body indwelling member. Therefore, it is possible to suppress kink since a gap is not generated between the contrast marker and a stent at an axial position. Bending rigidity of the contrast marker on the separating direction side is smaller than bending rigidity of the contrast marker on the side where the contrast marker comes close to the living body indwelling member. Therefore, even on the separating direction side relative to the contrast marker, it is possible to suppress kink of the delivery system by minimizing an excessive change in the bending rigidity which starts from the contrast marker. Accordingly, it is possible to improve insertion operability and safety.

When the delivery system is configured to further have a balloon that is disposed on an outer surface of the shaft portion so as to be inflatable, and that has the living body indwelling member mounted thereon so as to expand the living body indwelling member, the system having the balloon can suppress kink probably occurring in an end portion of the contrast marker.

When the bending rigidity of the contrast marker is configured to decrease with a gradient in the separating direction, the bending rigidity of the contrast marker is smoothly changed. Accordingly, it is possible to more effectively suppress kink occurring in the delivery system on the separating direction side of the contrast marker.

When the contrast marker is disposed on the proximal side of the living body indwelling member, it is possible to effectively suppress kink occurring on the proximal side of the stent on which bending stress is likely to act.

When the contrast marker is formed of a coil whose winding pitch is widened in the separating direction, it is possible to easily form the contrast marker whose bending rigidity is changed stepwise or with a gradient.

When contrast marker is configured to be a tubular member having at least one of a hole or a slit which is formed in order to decrease the bending rigidity, it is possible to easily form the contrast marker whose bending rigidity is changed stepwise or with a gradient.

When the living body indwelling member is configured to be a stent, the system having the stent can suppress kink probably occurring in an end portion of the contrast marker.

### Brief Description of Drawings

Fig. 1 is a plan view of a delivery system for a living body indwelling member according to an embodiment of the present invention.
Fig. 2 is a longitudinal sectional view of a distal portion of the delivery system for the living body indwelling member.
Fig. 3 is a plan view illustrating another example of a contrast marker.
Fig. 4 is a plan view illustrating further another example of the contrast marker.
Fig. 5 is a plan view illustrating further another example of the contrast marker.
Fig. 6 is a plan view illustrating further another example of the contrast marker.
Fig. 7 is a plan view illustrating further another example of the contrast marker.
Fig. 8 is a plan view illustrating further another example of the contrast marker.

### Description of Embodiments

Hereinafter, an embodiment according to the present invention will be described with reference to the drawings. In some cases, dimensional proportions in the drawings may be exaggerated and different from actual proportions for convenience of description.

A delivery system for a living body indwelling member 10 according to the present embodiment is used in order to treat stenosis appearing in blood vessels, biliary ducts, bronchial tubes, esophagi, urethrae, or other living body lumens. In this description, a side inserted into a lumen is referred to as a "distal end" or a "distal side", and an operating hand side is referred to as a "proximal end" or a "proximal side".

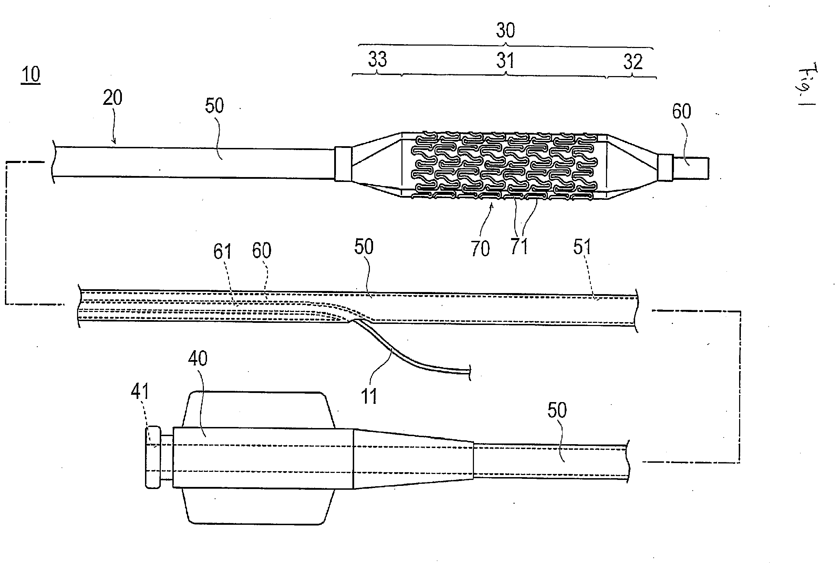

As illustrated in Fig. 1, the delivery system for the living body indwelling member 10 has an elongated shaft portion 20, a balloon 30 which is disposed on the distal portion of the shaft portion 20, a stent 70 (living body indwelling member) which is mounted on the balloon 30, and a hub 40 which is fixedly attached to the proximal end of the shaft portion 20.

The shaft portion 20 includes an outer tube 50 which is a tubular body whose distal end and proximal end are open, and an inner tube 60 which is arranged inside the outer tube 50. The outer tube 50 has a dilating lumen 51 in which a dilating fluid is circulated in order to inflate the balloon 30. The inner tube 60 has a guidewire lumen 61 into which a guidewire 11 is inserted. The dilating fluid may be either gas or liquid, and for example, a gas such as helium gas, CO₂ gas, O₂ gas, and the like, or a liquid such as a physiological salt solution, a contrast medium, and the like may be used.

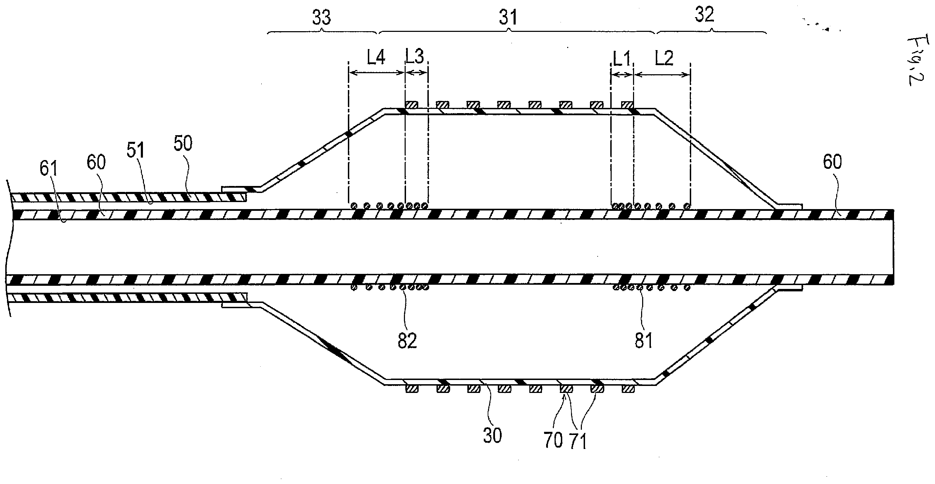

As illustrated in Fig. 2, the distal portion of the inner tube 60 penetrates the inside of the balloon 30, and is open on the further distal side relative to the balloon 30. As illustrated in Fig. 1, the proximal side thereof penetrates a side wall of the outer tube 50, and is fixedly attached to the outer tube 50 in a liquid-tight manner by using an adhesive or by means of heat sealing.

As illustrated in Fig. 1, the hub 40 includes a proximal opening portion 41 functioning as a port which communicates with the dilating lumen 51 of the outer tube 50 so as to circulate the dilating fluid. The proximal portion of the outer tube 50 is fixedly attached to the hub 40 in a liquid-tight manner by using an adhesive, by means of heat sealing, by using a fastener (not illustrated), or the like.

It is preferable to form the outer tube 50 and the inner tube 60 by using a material which is flexible to some degree. For example, as the material, polyolefin such as polyethylene, polypropylene, polybutene, ethylene-propylene copolymer, an ethylene-acetate vinyl copolymer, ionomer, or a mixture of two or more of these materials and the like, soft polyvinyl chloride resin, polyamide, polyamide elastomer, polyester, polyester elastomer, polyurethane, fluorine resin such as polytetrafluoroethylene, and the like, silicone rubber, latex rubber, or the like can be used.

As a configuration material of the hub 40, it is possible to preferably use polycarbonate, polyamide, polysulfone, polyacrylate, methacrylate-butylene-styrene copolymer, or the like.

The balloon 30 inflates so as to spread out stenosis, and then the dilating force of the balloon plastically deforms and expands the stent 70. The balloon 30 brings the stent 70 into close contact with an inner surface of a target area, and fixes the stent 70 to the inner surface. In order to efficiently spread out a predetermined range when the balloon 30 inflates, the balloon 30 has a cylindrical portion 31 which is formed in a substantially cylindrical shape in the axially central portion and which has substantially the same diameter. A first decreased diameter portion 32 whose diameter decreases in a tapered shape toward the distal side is disposed on the distal side of the cylindrical portion 31 of the balloon 30. A second decreased diameter portion 33 whose diameter decreases in a tapered shape toward the proximal side is disposed on the proximal side thereof.

The distal side of the first decreased diameter portion 32 is fixedly attached on an outer wall surface of the inner tube 60 in a liquid-tight manner by using an adhesive or by means of heat sealing, for example. The proximal side of the second decreased diameter portion 33 is fixedly attached on an outer wall surface of the distal portion of the outer tube 50 in a liquid-tight manner by using an adhesive or by means of heat sealing, for example. Therefore, the inside of the balloon 30 can communicate with the dilating lumen 51 formed in the outer tube 50, and the dilating fluid can flow into the inside of the balloon 30 from the proximal side via the dilating lumen 51. The balloon 30 is caused to inflate by the dilating fluid flowing into the balloon 30. The balloon 30 is brought into a folded state by discharging the dilating fluid flowing into the balloon 30.

It is preferable to form the balloon 30 by using a material which is flexible to some degree. For example, as the material, polyolefin such as polyethylene, polypropylene, polybutene, ethylene-propylene copolymer, ethylene-acetate vinyl copolymer, ionomer, or a mixture of two or more of these materials and the like, soft polyvinyl chloride resin, polyamide, polyamide elastomer, polyester, polyester elastomer, polyurethane, fluorine resin, silicone rubber, latex rubber, or the like can be used.

The stent 70 is a so-called balloon-expandable stent which plastically deforms and expands due to the inflating force of the balloon 30, and is mounted on the cylindrical portion 31 of the balloon 30 in a state where the stent 70 is contracted.

The stent 70 is configured to have a cylindrical shape as a whole. In the stent 70, multiple annular portions 71 in which wires are corrugated in a wavelike and formed in an annular shape are arranged in the axial direction and the mutually adjacent annular portions 71 are connected to each other. Then, the corrugated portions of the respective annular portions 71 are plastically deformed to spread out, thereby enabling the stent 70 to expand so as to increase the diameter thereof. Without being limited to the above-described configuration, the stent can employ a known configuration such as those which have a mesh shape, for example.

It is preferable to use metal which is biocompatible for the configuration material of the stent 70. For example, the configuration material includes an iron-based alloy such as stainless steel and the like, tantalum (tantalum alloy), platinum (platinum alloy), gold (gold alloy), a cobalt-based alloy such as a cobalt-chromium alloy and the like, a titanium alloy, a niobium alloy, or the like.

Then, a distal side marker 81 and a proximal side marker 82 which have X-ray contrasting property or ultrasound contrasting property are fixed on the inner tube 60.

The distal side marker 81 is arranged to extend in a direction toward the distal end where a part of the proximal side overlaps the distal portion of the contracted stent 70 at the axial position and where a part of the distal side does not overlap the stent 70. The distal side marker 81 is configured to include a coil around which wires having contrasting property are wound. A winding pitch (gap between the adjacent wires) is gradually narrowed toward the proximal side, and the winding pitch is gradually widened toward the distal side. Therefore, the bending rigidity of the distal side marker 81 decreases with a gradient in a distal end direction (separating direction) away from the stent 70. Preferably, the diameter of the distal side marker 81 is 0.5 mm to 1. 0 mm, but is not limited thereto. Preferably, a length L1 of a portion where the distal side marker 81 and the stent 70 overlap each other is 0 mm to 1.0 mm, but is not limited thereto. Preferably, a length L2 of a portion where the distal side marker 81 and the stent 70 do not overlap each other is 0.5 mm to 3.0 mm, but is not limited thereto. The expression of the length L1 of the overlapped portion = 0 mm means that an edge portion on the proximal side of the distal side marker 81 coincides with an edge portion on the distal side of the stent 70 at the axial position. This configuration is also included in the technical scope of the present invention.

The proximal side marker 82 is arranged to extend in a direction toward the proximal end where a part of the distal side overlaps the proximal portion of the contracted stent 70 at the axial position and where a part of the proximal side does not overlap the stent 70. The proximal side marker 82 is configured to include a coil around which wires having contrasting property are wound. The winding pitch is gradually narrowed toward the distal side, and the winding pitch is gradually widened toward the proximal side. Therefore, the bending rigidity of the proximal side marker 82 decreases with a gradient in a proximal end direction (separating direction) away from the stent 70. Preferably, the diameter of the proximal side marker 82 is 0.5 mm to 1.0 mm, but is not limited thereto. Preferably, a length L3 of a portion where the proximal side marker 82 and the stent 70 overlap each other is 0 mm to 1.0 mm, but is not limited thereto. Preferably, a length L4 of a portion where the proximal side marker 82 and the stent 70 do not overlap each other is 0.5 mm to 3.0 mm, but is not limited thereto. The expression of the length L3 of the overlapped portion = 0 mm means that an edge portion on the distal side of the proximal side marker 82 coincides with an edge portion on the proximal side of the stent 70 at the axial position. This configuration is also included in the technical scope of the present invention.

As the X-ray contrast marker, the material of the distal side marker 81 and the proximal side marker 82 is preferably formed from one metal or two or more alloys which include at least any one of a group containing gold, platinum, iridium, tungsten, an alloy of these materials, or silver-palladium alloy. As the ultrasound contrast marker, it is possible to use stainless steel or the like in addition to the above-described metal.

Next, an operation of the delivery system for the living body indwelling member 10 according to the present embodiment will be described as an example of a case where the delivery system is inserted into the blood vessel in order to treat stenosis.

First, before the stenosis in the blood vessel is treated, air inside the balloon 30 and the dilating lumen 51 is removed as much as possible. The air inside the balloon 30 and the dilating lumen 51 is replaced with the dilating fluid. At this time, the balloon 30 is in a folded state.

Next, a sheath is caused to indwell the blood vessel of a patient by means of a Seldinger technique, for example. In a state where the guidewire 11 is inserted into the guidewire lumen 61, the guidewire 11 and the delivery system for the living body indwelling member 10 are inserted into the blood vessel through the inside of the sheath. Subsequently, while the guidewire 11 is caused to move in advance, the delivery system for the living body indwelling member 10 is moved forward so that the balloon 30 reaches stenosis.

At this time, a portion on the proximal side of the distal side marker 81 overlaps the distal portion of the stent 70 at the axial position, and the distal side marker 81 is brought into a state of entering the distal portion of the stent 70, or into a state of coinciding with the edge portion of the distal portion of the stent 70. Accordingly, there exists no portion interposed between the hard stent 70 and the distal side marker 81 at the axial position (specifically, a portion of only the balloon 30 and the inner tube shaft 60). Therefore, in a case where bending stress acts, for example, in a case where the delivery system for the living body indwelling member 10 passes through the meandering peripheral blood vessel or the like, kink of the delivery system for the living body indwelling member 10 is suppressed between the stent 70 and the distal side marker 81. Furthermore, the bending rigidity of the portion where the distal side marker 81 and the stent 70 do not overlap each other is decreased toward the distal side. Accordingly, an excessive change in the bending rigidity which starts from the distal side marker 81 is minimized on the further distal side relative to the distal side marker 81, thereby suppressing the kink of the delivery system for the living body indwelling member 10.

Furthermore, a portion on the distal side of the proximal side marker 82 overlaps the proximal portion of the stent 70 at the axial position, and the proximal side marker 82 is brought into a state of entering the proximal portion of the stent 70, or into a state of coinciding with the edge portion on the proximal side of the stent 70. Accordingly, there exists no portion interposed between the hard stent 70 and the proximal side marker 82 at the axial position (specifically, a portion of only the balloon 30 and the inner tube shaft 60) . Therefore, in a case where bending stress acts, for example, in a case where the delivery system for the living body indwelling member 10 passes through the meandering peripheral blood vessel or the like, kink of the delivery system for the living body indwelling member 10 is suppressed between the stent 70 and the proximal side marker 82. Furthermore, the bending rigidity of the portion where the proximal side marker 82 and the stent 70 do not overlap each other is decreased in a direction toward the proximal end portion. Accordingly, an excessive change in the bending rigidity which starts from the distal side marker 81 is minimized on the further proximal side relative to the proximal side marker 82, thereby suppressing the kink of the delivery system for the living body indwelling member 10. The bending stress is more likely to act in the vicinity of the proximal side marker 82 than in the vicinity of the distal side marker 81. Accordingly, a particularly excellent advantageous effect is expected.

In this manner, the distal side marker 81 and the proximal side marker 82 suppress the kink. Accordingly, it is possible to improve insertion operability of the delivery system for the living body indwelling member 10. Unexpected spreading of the stent 70 which is caused by the kink is suppressed. Therefore, it is possible to prevent the stent 70 from slipping out therefrom, thereby improving safety.

Next, in a state where the balloon 30 is located in the stenosis, a predetermined amount of the dilating fluid is injected through the proximal opening portion 41 of the hub 40 by using an indeflator, a syringe, a pump, or the like. The dilating fluid is fed into the balloon 30 through the dilating lumen 51, thereby expanding the folded balloon 30. In this manner, the cylindrical portion 31 of the balloon 30 spreads out the stenosis, and plastically deforms and spreads out the stent 70 mounted on the outer periphery of the balloon 30. Accordingly, it is possible to satisfactorily maintain a state where the stenosis is spread out by the stent 70.

Thereafter, the dilating fluid is aspirated and discharged through the proximal opening portion 41, and the balloon 30 is brought into a deflated and folded state. At this time, the stent 70 is caused to indwell the stenosis while the stent 70 remains in an expanded state. Thereafter, the guidewire 11 and the shaft portion 20 are removed from the blood vessel via the sheath. In this manner, procedures are completed.

As described above, the delivery system for a living body indwelling member 10 according to the present embodiment includes the shaft portion 20 that extends in a long length, the stent 70 (living body indwelling member) that is disposed on the distal side of the shaft portion 20, and that is caused to indwell the living body, and the distal side marker 81 (contrast marker) and the proximal side marker 82 (contrast marker) that are respectively disposed on the proximal side and the distal side of the stent 70, and that have contrasting property. The distal side marker 81 and the proximal side marker 82 are arranged up to the position where the contrast markers do not overlap the stent 70 in the separating direction away from the stent 70 from the position where the contrast markers overlap the stent 70 at the axial position of the shaft portion 20, or from the position where the contrast markers coincide with the edge portion of the stent 70. The bending rigidity of the contrast markers on the separating direction side is smaller than the bending rigidity of the contrast markers on the side where the contrast markers come close to the stent 70. Therefore, without forming a gap in the axial direction, kink can be suppressed between the distal side marker 81 and the stent 70 and between the proximal side marker 82 and the stent 70. An excessive change in the bending rigidity is minimized on the distal side of the distal side marker 81 and the proximal side of the proximal side marker 82. Accordingly, it is possible to suppress the kink occurring in these areas. In this manner, it is possible to improve insertion operability and safety of the delivery system for the living body indwelling member 10.

In addition, the delivery system for the living body indwelling member 10 further has the balloon 30 which is disposed on the outer surface of the shaft portion 20 so as to be inflatable and which has the stent 70 mounted thereon so as to expand the stent 70. Accordingly, the system having the balloon 30 can suppress kink probably occurring at the end portion of the distal side marker 81 and the proximal side marker 82.

In addition, the bending rigidity of the distal side marker 81 decreases with a gradient in the direction toward the distal end, and the bending rigidity of the proximal side marker 82 decreases with a gradient in the direction toward the proximal end. Accordingly, by smoothly changing the bending rigidity, it is possible to more effectively suppress kink occurring on the distal side of the distal side marker 81 and the proximal side of the proximal side marker 82.

In addition, the proximal side marker 82 is disposed on the proximal side of the stent 70. Accordingly, it is possible to effectively suppress kink occurring on the proximal side of the stent 70 on which bending stress is likely to act.

In addition, the distal side marker 81 is formed of the coil whose winding pitch is widened in the direction toward the distal end, and the proximal side marker 82 is formed of the coil whose winding pitch is widened in the direction toward the proximal end. Accordingly, it is possible to easily form a marker whose bending rigidity varies with a gradient.

Without being limited only to the above-described embodiment, the present invention can be modified in various ways by those skilled in the art within the technical idea of the present invention. For example, there may be provided only one of the distal side marker 81 or the proximal side marker 82 which are contrast markers.

In addition, without being limited to the stent 70, for example, the living body indwelling member which is caused to indwell the living body may be an ASD closure which is caused to indwell an atrial septal defect (ASD) in order to close the ASD, a vascular filter which is caused to indwell the blood vessel in order to remove a thrombus and the like which are mixed in the blood, or the like. When the living body indwelling member is caused to indwell the living body, if the balloon is not useful, the delivery system for the living body indwelling member may not include the balloon.

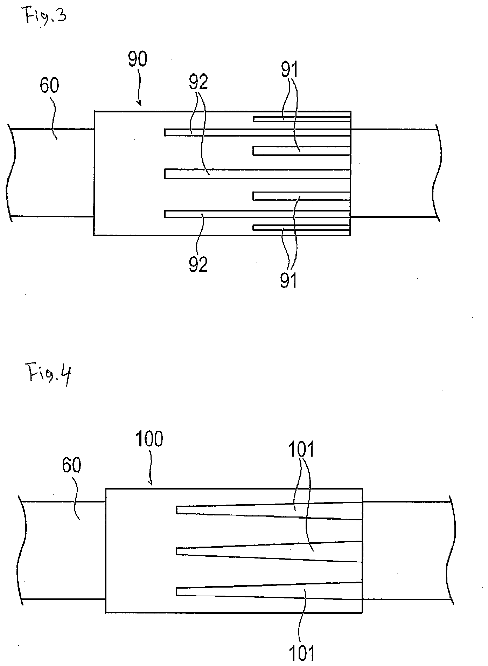

In addition, as in the modification example illustrated in Fig. 3, bending rigidity may be changed by forming slits 91 and 92 extending in the axial direction in a contrast marker 90 which is formed of a tubular member. In this case, the bending rigidity can be changed stepwise by forming the multiple slits 91 and 92 which are different from each other in length. In addition, as illustrated in Fig. 4, the bending rigidity can be changed with a gradient by changing a width of a slit 101 with a gradient in the axial direction. The contrast marker which is formed of this tubular member can be attached to the inner tube 60 by forming the contrast marker, for example, in a half-split shape (discontinuous shape in the circumferential direction) having a cutout portion extending in the axial direction, and by deforming the cutout portion so as to be closed after covering the inner tube 60 from the cutout portion.

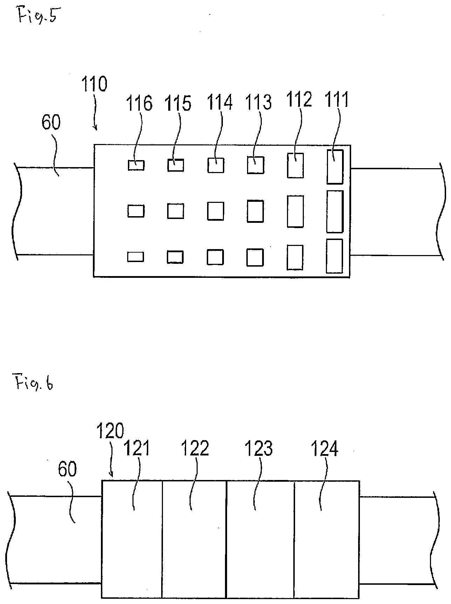

In addition, as in another modification example illustrated in Fig. 5, the bending rigidity may be changed by forming multiple hole portions 111 to 116 in a contrast marker 110 which is formed of a tubular member. In this case, the bending rigidity can be changed stepwise or with a gradient by changing a size, a shape, the number and the like of holes in accordance with the axial position of the contrast marker 110.

In addition, as in further another modification example illustrated in Fig. 6, a contrast marker 120 may be formed in such a way that multiple sections 121, 122, 123, and 124 having mutually different bending rigidities are joined to one another side by side in the axial direction. In this case, for example, the bending rigidity can be changed stepwise in such a way that the first section 121 contains 99% platinum, the second section 122 includes an alloy containing 80% platinum and 20% copper softer than the platinum (lower modulus of longitudinal elasticity), and the third section 123 and the fourth section 124 include a copper alloy in which platinum content is further reduced stepwise. The number of sections having mutually different bending rigidities is not particularly limited. In addition, the bending rigidity can be changed with a gradient by changing a material mixing ratio in a stepless manner (with a gradient) along the axial direction of the contrast marker. In addition, a type of material to be mixed therewith is not particularly limited as long as contrasting property can be shown when being mixed. Therefore, for example, a particulate contrast material may be mixed with a resin.

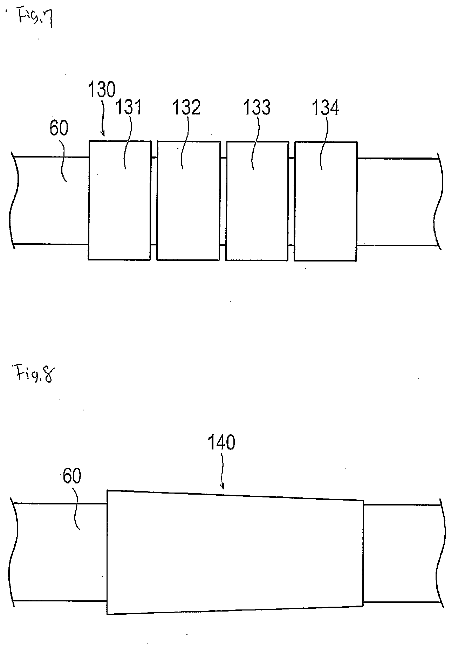

In addition, as in further another modification example illustrated in Fig. 7, a contrast marker 130 may be formed in such a way that multiple sections 131, 132, 133, and 134 having mutually different bending rigidities are not joined to one another and are arranged side by side in the axial direction. In this case, a gap may be disposed or may not be disposed between the sections which are adjacent to each other.

In addition, as in further another modification example illustrated in Fig. 8, bending rigidity can be changed stepwise or with a gradient by changing the thickness of a contrast marker 140 along the axial direction.

### Reference Signs List

- 10: DELIVERY SYSTEM FOR LIVING BODY INDWELLING MEMBER,
- 20: SHAFT PORTION,
- 30: BALLOON,
- 50: OUTER TUBE,
- 60: INNER TUBE,
- 70: STENT (LIVING BODY INDWELLING MEMBER),
- 81: DISTAL SIDE MARKER (CONTRAST MARKER),
- 82: PROXIMAL SIDE MARKER (CONTRAST MARKER),
- 90, 100, 110, 120, 130, 140: CONTRAST MARKER.

## Claims

1. A delivery system (10) for a living body indwelling member (70), comprising:
a shaft portion (20) that extends in a long length;
a living body indwelling member (70) that is disposed on a distal side of the shaft portion (20), and that is caused to indwell a living body; and
a contrast marker (81, 82) that is disposed on at least one side of a proximal side or a distal side of the living body indwelling member (70), and that has contrasting property,
wherein the contrast marker (81, 82) is arranged up to a position where the contrast marker (81, 82) does not overlap the living body indwelling member (70) in a separating direction away from the living body indwelling member (70) from a position where the contrast marker (81, 82) overlaps the living body indwelling member (70) at an axial position of the shaft portion (20), or from a position where the contrast marker (81, 82) coincides with an edge portion of the living body indwelling member (70),
**characterized in that** bending rigidity of the contrast marker (81, 82) on the separating direction side is smaller than bending rigidity of the contrast marker (81, 82) on a side where the contrast marker (81, 82) comes close to the living body indwelling member (70).

2. The delivery system (10) for a living body indwelling member (70) according to Claim 1, further comprising:
a balloon (30) that is disposed on an outer surface of the shaft portion (20) so as to be inflatable, and that has the living body indwelling member (70) mounted there on so as to expand the living body indwelling member (70).

3. The delivery system (10) for a living body indwelling member (70) according to Claim 1 or 2,
wherein the bending rigidity of the contrast marker (81, 82) decreases with a gradient in the separating direction.

4. The delivery system (10) for a living body indwelling member (70) according to anyone of Claims 1 to 3,
wherein the contrast marker (81, 82) is disposed on the proximal side of the living body indwelling member (70).

5. The delivery system (10) for a living body indwelling member (70) according to anyone of Claims 1 to 4,
wherein the contrast marker (81, 82) is formed of a coil whose winding pitch is widened in the separating direction.

6. The delivery system (10) for a living body indwelling member (70) according to anyone of Claims 1 to 4,
wherein the contrast marker (81, 82) is a tubular member having at least one of a hole or a slit (91, 92, 101) which is formed in order to decrease the bending rigidity.

7. The delivery system (10) for a living body indwelling member (70) according to anyone of Claims 1 to 6,
wherein the living body indwelling member (70) is a stent.

## Patentansprüche

1. Einführsystem (10) für ein in einem lebenden Körper verbleibendes Element (70), umfassend:
einen Schaftabschnitt (20), der sich in einer Längsrichtung erstreckt;
ein in einem lebenden Körper verbleibendes Element (70), das sich auf einer distalen Seite des Schaftabschnitts (20) befindet und das in einem lebenden Körper verbleiben soll; und
einen Kontrastmarker (81, 82), der sich auf wenigstens einer von einer proximalen Seite und einer distalen Seite des in einem lebenden Körper verbleibenden Elements (70) befindet und der eine kontrastierende Eigenschaft besitzt,
wobei der Kontrastmarker (81, 82) bis zu einer Position angeordnet ist, wo der Kontrastmarker (81, 82) ausgehend von einer Position, wo der Kontrastmarker (81, 82) das in einem lebenden Körper verbleibende Element (70) an einer axialen Position des Schaftabschnitts (20) überlappt, oder ausgehend von einer Position, wo der Kontrastmarker (81, 82) mit einem Randabschnitt des in einem lebenden Körper verbleibenden Elements (70) zusammenfällt, das in einem lebenden Körper verbleibende Element (70) in einer Trennrichtung weg von dem in einem lebenden Körper verbleibenden Element (70) nicht überlappt,
**dadurch gekennzeichnet, dass** die Biegesteifigkeit des Kontrastmarkers (81, 82) auf der Seite der Trennrichtung geringer ist als die Biegesteifigkeit des Kontrastmarkers (81, 82) auf einer Seite, wo sich der Kontrastmarker (81, 82) nahe bei dem in einem lebenden Körper verbleibenden Element (70) befindet.

2. Einführsystem (10) für ein in einem lebenden Körper verbleibendes Element (70) nach Anspruch 1, ferner umfassend:
einen Ballon (30), der sich auf einer Außenseite des Schaftabschnitts (20) befindet, so dass er aufblasbar ist, und an dem das in einem lebenden Körper verbleibende Element (70) angebracht ist, um das in einem lebenden Körper verbleibende Element (70) aufzuweiten.

3. Einführsystem (10) für ein in einem lebenden Körper verbleibendes Element (70) nach Anspruch 1 oder 2,
wobei die Biegesteifigkeit des Kontrastmarkers (81, 82) mit einem Gradienten in der Trennrichtung abnimmt.

4. Einführsystem (10) für ein in einem lebenden Körper verbleibendes Element (70) nach einem der Ansprüche 1 bis 3,
wobei sich der Kontrastmarker (81, 82) auf der proximalen Seite des in einem lebenden Körper verbleibenden Elements (70) befindet.

5. Einführsystem (10) für ein in einem lebenden Körper verbleibendes Element (70) nach einem der Ansprüche 1 bis 4,
wobei der Kontrastmarker (81, 82) aus einer Spule gebildet ist, deren Wicklungssteigung in der Trennrichtung breiter wird.

6. Einführsystem (10) für ein in einem lebenden Körper verbleibendes Element (70) nach einem der Ansprüche 1 bis 4,
wobei der Kontrastmarker (81, 82) ein rohrförmiges Element mit mindestens einem Loch oder einem Schlitz (91, 92, 101) ist, das/der dazu ausgebildet ist, die Biegesteifigkeit herabzusetzen.

7. Einführsystem (10) für ein in einem lebenden Körper verbleibendes Element (70) nach einem der Ansprüche 1 bis 6,
wobei das in einem lebenden Körper verbleibende Element (70) ein Stent ist.

## Revendications

1. Système de mise en place à demeure (10) d'un élément (70) dans un corps vivant, comprenant :
une partie tige (20) qui s'étend dans le sens de la longueur ;
un élément (70) mis en place à demeure dans un corps vivant qui est disposé sur un côté distal de la partie tige (20), et qui est amené à être placé à demeure dans un corps vivant ; et
un marqueur de contraste (81, 82) qui est situé sur au moins un côté d'un côté proximal ou d'un côté distal de l'élément (70) mis en place à demeure dans un corps vivant, et qui présente une propriété de contraste,
dans lequel le marqueur de contraste (81, 82) est placé dans une position où le marqueur de contraste (81, 82) ne se superpose pas à l'élément (70) mis en place à demeure dans un corps vivant dans une direction de séparation par rapport à l'élément (70) mis en place à demeure dans un corps vivant depuis une position où le marqueur de contraste (81, 82) se superpose à l'élément (70) mis en place à demeure dans un corps vivant au niveau d'une position axiale de la partie tige (20), ou depuis une position où le marqueur de contraste (81, 82) coïncide avec une partie de bord de l'élément (70) mis en place à demeure dans un corps vivant,
**caractérisé en ce que** la rigidité en flexion du marqueur de contraste (81, 82) du côté de la direction de séparation est inférieure à la rigidité en flexion du marqueur de contraste (81, 82) d'un côté où le marqueur de contraste (81, 82) devient proche de l'élément (70) mis en place à demeure dans un corps vivant.

2. Système de mise en place à demeure (10) d'un élément (70) dans un corps vivant selon la revendication 1, comprenant en outre :
un ballonnet (30) qui est disposé sur une surface extérieure de la partie tige (20) de manière à pouvoir être gonflé, et sur lequel est monté l'élément (70) mis en place à demeure dans un corps vivant de manière à déployer l'élément (70) mis en place à demeure dans un corps vivant.

3. Système de mise en place à demeure (10) d'un élément (70) dans un corps vivant selon la revendication 1 ou 2,
dans lequel la rigidité en flexion du marqueur de contraste (81, 82) diminue de gradient dans la direction de séparation.

4. Système de mise en place à demeure (10) d'un élément (70) dans un corps vivant selon l'une quelconque des revendications 1 à 3,
dans lequel le marqueur de contraste (81, 82) est disposé sur le côté proximal de l'élément (70) mis en place à demeure dans un corps vivant.

5. Système de mise en place à demeure (10) d'un élément (70) dans un corps vivant selon l'une quelconque des revendications 1 à 4,
dans lequel le marqueur de contraste (81, 82) est formé d'une bobine dont le pas d'enroulement est élargi dans la direction de séparation.

6. Système de mise en place à demeure (10) d'un élément (70) dans un corps vivant selon l'une quelconque des revendications 1 à 4,
dans lequel le marqueur de contraste (81, 82) est un élément tubulaire ayant au moins un orifice ou une fente (91, 92, 101) qui est formé de manière à diminuer la rigidité en flexion.

7. Système de mise en place à demeure (10) d'un élément (70) dans un corps vivant selon l'une quelconque des revendications 1 à 6,
dans lequel l'élément (70) mis en place à demeure dans un corps vivant est un stent.
